# EUROPÄISCHE PATENTSCHRIFT

(11) **EP 1 209 156 B1**
(45) Veröffentlichungstag und Bekanntmachung des Hinweises auf die Patenterteilung: **11.05.2005**
(21) Anmeldenummer: 01130402.9
(22) Anmeldetag: 29.06.1996
(51) Int. Cl.: B01D 3/10, B01J 19/32, B01D 3/00, C07D 311/72

(54) **Verfahren zur Gewinnung von reinem Vitamin-E-acetat oder reinen Tocopherolen durch Rektifikation zwecks Reinigung von leichter siedenden und höher siedenden Verunreinigungen**
Process for extracting pure vitamin E-acetate or pure tocopheroles by rectification to remove lower and higher boiling contaminants
Procédé d extraction de l acétate de la vitamine E pur ou des tocophéroles pures par rectification pour enlever des substances contaminantes à point d ébullition supérieure et inférieures

(30) Priorität: 08.07.1995 DE 19524928
(43) Veröffentlichungstag der Anmeldung: 29.05.2002
(62) Teilanmeldung aus: 96924820.2
(73) Patentinhaber: BASF AKTIENGESELLSCHAFT, 67056 Ludwigshafen (DE)
(72) Erfinder: Hartmann, Horst, 67459 Böhl-Iggelheim (DE); Burst, Wolfram, 68199 Mannheim (DE); Kaiser, Wulf, Dr., 6612 Ascona (CH); Laas, Harald, Dr., 67133 Maxdorf (DE); Grafen, Paul, Dr., 67273 Weisenheim (DE); Bockstiegel, Bernhard, Dr., 67354 Römerberg (DE); Baldenius, Kai-Uwe, Dr., 67063 Ludwigshafen (DE)

(56) Entgegenhaltungen:
- EP-A- 0 467 395
- WO-A-95/04731
- DE-B- 1 088 026
- DE-B- 1 110 613
- FR-A- 1 575 344
- FR-A- 2 212 295
- US-A- 2 047 444

## Beschreibung

Die Erfindung betrifft ein Verfahren zur Gewinnung von reinem Vitamin-E-acetat oder reinen Tocopherolen durch Rektifikation zwecks Reinigung von leichter siedenden und höher siedenden Verunreinigungen.

Bei der destillativen Aufarbeitung von Produktgemischen erzielt man im allgemeinen die besten Ergebnisse bei der sogenannten Gegenstromdestillation (auch Rektifikation genannt), d.h. einem speziellen Destillationsverfahren mit Gegenstromführung von in Rektifizierkolonnen abströmendem Rücklauf und aufströmendem Dampf. Üblicherweise arbeitet man dabei mit Rektifizierkolonnen, bei denen das zu trennende Gemisch im mittleren Teil der Kolonne eingebracht wird, der Dampf sich auf seinem Weg durch die Kolonne von unten nach oben an leichterflüchtigen Komponenten anreichert, während sich der Rücklauf von oben nach unten an schwerer flüchtigen Komponenten anreichert. Der Stoff- und Wärmetransport wird durch in die Kolonne eingebaute Elemente, wie Kolonnenböden, Füllkörper oder Packungen, die für eine ausreichende Berührungszeit der Phasen und eine hinreichende große Phasengrenzfläche sorgen, intensiviert. Durch diese Kolonneneinbauten, verbunden mit dem abströmenden Rücklauf ergibt sich jedoch in der Kolonne ein Widerstand, der sogenannte Druckverlust. Der Druckverlust einer Kolonne hängt neben Art und Menge der zu rektifizierenden Verbindungen sehr stark von der Art der Kolonneneinbauten ab.

Für die Auftrennung von hochsiedenden Substanzgemischen, die eine hohe Trennleistung erfordern, verwendet man im allgemeinen Rektifizierkolonnen, die in regelmäßiger Geometrie systematisch aufgebaute Packungen mit definierten Durchtrittsbereichen für die Gegenstromphasen aufweisen, da sich regelmäßig strukturierte Packungen gegenüber Füllkörpern durch höhere Belastbarkeit und eine bessere Trennwirkung auszeichnen, einen geringeren spezifischen Druckverlust sowie ein geringeres erforderliches Packungsvolumen und daher auch eine kleinere notwendige Stoff- und wärmeaustauschende Höhe aufweisen. Sie werden daher bei allen Vakuumrektifikationen eingesetzt, bei denen es wegen der Temperaturempfindlichkeit des zu trennenden Gemisches besonders auf eine Begrenzung des Kolonnendruckverlustes ankommt. Besonders geeignete Kolonnenpackungen sind Metallgewebepackungen vom Typ BX und CY der Firma Sulzer (vgl. Firmenschrift "Trennkolonnen für Destillation und Absorption" der Firma Sulzer) und ähnlich wirksame Metallgewebepackungen anderer Firmen, wie der Montz GmbH.

Eine schematische Darstellung solcher Kolonnen findet man beispielsweise auf Seite 103 des Lehrbuches "Thermische Trennverfahren" von Klaus Sattler, VCH Verlagsges.mbH, Weinheim (BRD), 1988. Bezüglich näherer Einzelheiten über die Rektifikation von Substanzgemischen verweisen wir auf dieses Lehrbuch von Klaus Sattler, Seiten 101-225, insbesondere 120-160 und 199-214.

Die höchste Produkttemperatur tritt im Sumpf einer Kolonne auf. Sie wird außer vom Kopfdruck maßgeblich von dem für die erforderliche Trennleistung bedingten Druckverlust der Kolonneneinbauten bestimmt. Bei Rektifikationskolonnen hat die Absenkung des Kopfdruckes auf unter 0,5 mbar keinen wesentlichen Einfluß auf die Sumpftemperatur.

Für zahlreiche hochsiedende Gemische ist die thermische Belastbarkeit so gering, daß sich trotz Verwendung der beschriebenen Metallgewebepackungen mit geordneter Struktur und Kopfdrucken in der Kolonne von nur 0,5 bis 1 mbar durch den Druckverlust an den für die erforderliche Trennleistung benötigten Gewebepackungen im Sumpf Temperaturen auftreten würden, die oberhalb des Zersetzungsbereichs der zu trennenden Verbindungen liegt. Für die destillative Auftrennung solcher Gemische arbeitet man daher bisher im allgemeinen im Hochvakuumbereich (ca. 10⁻¹ bis 10⁻⁵ mbar), d.h. man wendet Kurzwegdestillationen oder Molekulardestillationen an. Bei Gemischen mit geringen relativen Flüchtigkeiten sind mit diesen Destillationen hohe Reinheiten jedoch nur mit niedrigen Destillationsausbeuten erzielbar.

Ein Beispiel für ein Gemisch hochsiedender und stark luft- und/oder temperaturempfindlicher Substanzen, die eine hohe Trennleistung erfordern, ist synthetisches Vitamin-E-acetat (VEA), welches technisch durch Umsetzen von Trimethylhydrochinon mit Phytol oder Isophytol und anschließende Veresterung mit Acetanhydrid hergestellt wird und noch geringe Mengen an gefärbten leichter siedenden und höher siedenden Verunreinigungen enthält. Da VEA in zunehmendem Maße für die menschliche Ernährung bzw. Gesundheitsprophylaxe verwendet wird, werden immer höhere Anforderungen an die Reinheit dieses Produktes gestellt. Die für die Reinigung von Produkten im industriellen Maßstab im allgemeinen sehr vorteilhafte Rektifikation bereitet bei VEA durch dessen hohen Siedepunkt verbunden mit seiner Zersetzlichkeit bei höheren Temperaturen große Probleme. Deshalb werden bisher im wesentlichen Destillationen im Hochvakuum oder gar Molekulardestillationen durchgeführt um VEA bei möglichst niedrigen Temperaturen destillieren zu können.

Trotz Anwendung von Hochvakuum (10⁻¹ bis 10⁻⁵ mbar) werden nach dem Stand der Technik im allgemeinen nur Reinheiten von 97,3 % (vgl. DE 2 743 920), 98 % (vgl. DE 42 08 477 und JP-B-58 011 869), 98,5 % (vgl. US 3 459 773) oder 98,5 bis 99 % (vgl. DE 2 160 103) erzielt. Reinheiten über 99 % wurden nur durch Molekulardestillation erzielt, nämlich Reinheiten von 99,3 % gemäß JP-A 51/14671 und 99,5 % gemäß JP-A-62/226976, wobei anzumerken ist, daß vermutlich die so erhaltenen Produkte bei Untersuchung mit den heute angewendeten genaueren Analysenmethoden und reineren Vergleichssubstanzen niedrigere Reinheitswerte ergeben würden. Zudem sind die so erzielbaren Destillationsausbeuten jeweils recht gering.

Da jedoch Destillationen unter Hochvakuum insbesondere aber Molekulardestillationen, bei hohen Reinheiten neben dem Nachteil der geringen Destillationsausbeuten sowohl bezüglich der Investitionskosten als auch hinsichtlich der laufenden Betriebskosten außerordentlich aufwendig und daher sehr teuer sind, war es die Aufgabe der Erfindung, ein Verfahren zur destillativen Reinigung von Vitamin-E-acetat oder Tocopherolen zu entwickeln, bei dem keine Hochvakuumdestillation oder gar Molekulardestillation notwendig sind, d.h. ein Verfahren bei dem ein Kopfvakuum von 0,2 bis 2 mbar ausreicht und dadurch wesentlich weniger aufwendige Vakuumerzeugungsverfahren notwendig sind und eine hohe Destillationsausbeute erzielt wird.

Insbesondere war es die Aufgabe der Erfindung, ein Verfahren zur destillativen Feinreinigung von VEA zu finden, mit dessen Hilfe man farbloses VEA mit einer Reinheit von 99 % oder mehr und guten Destillationsausbeuten auch durch Rektifikation im Feinvakuum in Kolonnen enthaltend Metallgewebepackungen mit geordneter Struktur erhalten kann.

Gegenstand der Erfindung ist nun ein Verfahren zur Gewinnung von einem Vitamin-E-acetat oder reinen Tocopherolen aus Gemischen hochsiedender luft- und/oder temperaturempfindlicher Substanzen, die eine hohe Trennleistung erfordern, durch Rektifikation im Feinvakuum in Kolonnen enthaltend Metallgewebepackungen mit geordneter Struktur bei einem Kopfdruck von 0,2 bis 2 mbar, indem man die Rektifikation in einer Stoffaustauschkolonne durchführt, in der
a) die Flüssigkeitsverteilung mit Kanalverteilern mit gleich oder mehr als 500 Abtropfstellen/m², vorzugsweise 900 bis 1200 Abtropfstellen/m², vorgenommen wird,
b) in der die Kanäle der Verteiler im Winkel von etwa 90° um die Kolonnenachse verdreht zu den Gewebelagen der direkt unter dem Verteiler befindlichen Packungselemente angeordnet sind,
c) in der sich direkt unterhalb der Flüssigkeitsverteiler jeweils 2 oder mehrere Packungselemente mit einer Höhe von nur 20 bis 100 mm Höhe befinden, deren Gewebelagen jeweils um 90° um die Kolonnenachse zueinander gedreht sind,
d) daß die Kolonne so gestaltet ist, daß während der Rektifikation praktisch kein Wärmeaustausch durch die Kolonnenwand stattfinden kann,
e) daß bei luftempfindlichen Substanzen die Kolonne so gestaltet ist, daß praktisch unter Luftausschluß gearbeitet werden kann und
f) der Druckverlust pro Trennstufe durch eine erniedrigte Einstellung der Flüssigkeitsbelastung soweit erniedrigt wird, daß die im Sumpf auftretenden Temperaturen unterhalb des Zersetzungsbereichs von Vitamin-E-acetat erniedrigt werden.

Mit besonderem Vorteil gelingt das erfindungsgemäße Verfahren wenn man mit Gewebepackungen arbeitet, bei denen der Neigungswinkel der Zahnung der einzelnen Gewebelagen der Packung gegen die Kolonnenachse möglichst gering ist, um den spezifischen Druckverlust der Packung zu minimieren.

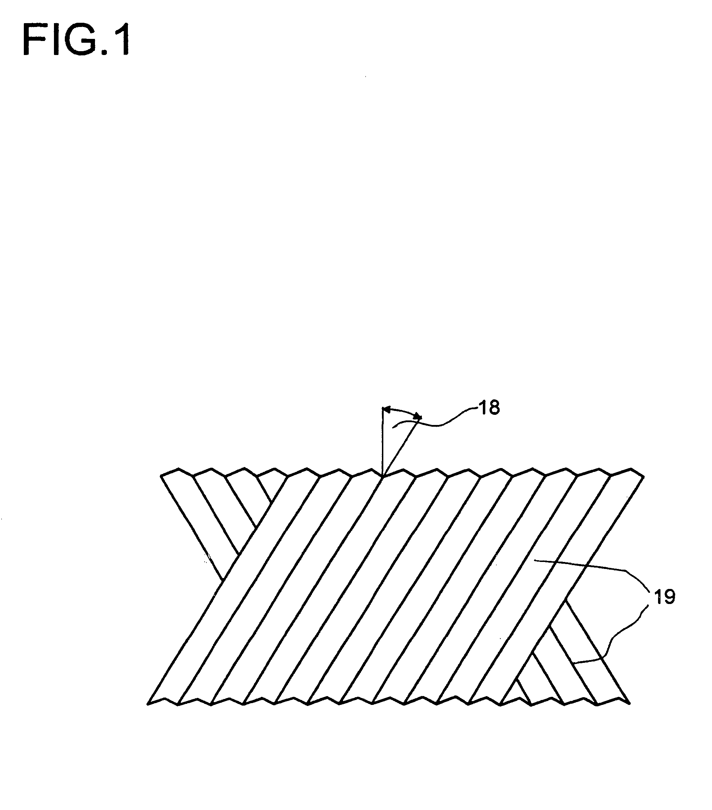

Während der Neigungswinkel der Zahnung bei den üblichen BX-Sulzerpackungen im allgemeinen 30° beträgt, werden bei dem erfindungsgemäßen Verfahren Gewebepackungen bevorzugt, deren Neigungswinkel der Zahnung der Gewebelagen gegen die Kolonnenachse 0 bis 25°, vorzugsweise 3 bis 10° beträgt. Der Neigungswinkel der Zahnung der Gewebelagen gegen die Kolonnenachse wird in Figur 1 veranschaulicht. In dieser Figur bedeutet 18 den Neigungswinkel der Zahnung der Gewebelagen gegen die Kolonnenachse und 19 Gewebelagen.

Besondere Bedeutung hat das erfindungsgemäße Verfahren für die Feinreinigung von VEA, d.h. für den Fall, daß man als Gemisch ein mit gefärbten leichter siedenden und höher siedenden Substanzen verunreinigtes VEA rektifiziert.

Am Beispiel der Reinigung von VEA soll im folgenden das erfindungsgemäße Verfahren mit seinen apparativen und verfahrenstechnischen Entwicklungen näher erläutert werden.

Bei Betriebskolonnen sind Kopfdrucke von 0,5 bis 1 mbar noch mit vertretbarem technischem Aufwand realisierbar. VEA weist bei einem Druck von 1 mbar eine Siedetemperatur von ca. 240°C auf. Durch die begrenzte thermische Stabilität von VEA ist die Temperatur im Kolonnensumpf auf ca. 260 bis 270°C begrenzt. Eine Rektifikationskolonne darf daher maximal mit einem Sumpfdruck von nur 4 mbar betrieben werden. Dies bedeutet, daß nur ein Druckverlust von ca. 3 bis 3,5 mbar zwischen Kopf und Sumpf der Kolonne toleriert werden kann. Dies ist sehr schwierig zu erreichen, da zur Feinreinigung von VEA eine Trennleistung von etwa 10 bis 30 Trennstufen erforderlich ist und man normalerweise mit einem Druckverlust von 0,3 bis 0,5 mbar pro Trennstufe rechnen muß. In den erfindungsgemäßen Arbeitsbereichen lagen bezüglich der Druckverluste keine Messungen vor.

Gemäß Merkmal a) des Hauptanspruch wird eine Flüssigkeitsverteilung mit Kanalverteilern mit gleich oder mehr als 500 Abtropfstellen beansprucht. Ähnliche auch "Kapillarverteiler" genannte aber runde Verteiler werden von den Firmen Sulzer und Montz vertrieben und werden beispielsweise in EP 512 277 beschrieben. Bekannte Kanalverteiler weisen im allgemeinen nur 50 bis 60 Abtropfstellen pro m² auf.

Die erfindungsgemäße Verwendung der Kanalverteiler bewirkt auf 2 verschiedene Weisen eine Verminderung des sogenannten Druckverlustes. Zum einen bedingen sie eine schnelle äußerst feine Verteilung und damit letztlich eine bessere Nutzung der Packung zum Verteilen des zu trennenden Gemisches und zum anderen eine sehr geringe Berieselungsdichte. Für Sulzer-Packungen vom BX-Typ werden Flüssigkeitsbelastungen ab ca. 0,2 m³/m²·h als untere Belastungsgrenze angegeben. Durch die erfindungsgemäße Anwendung der Kanalverteiler mit gleich oder mehr als 500, vorzugsweise 900 bis 1200 Abtropfstellen werden beim VEA Flüssigkeitsbelastungen beim erfindungsgemäßen Verfahren von nur 0,03 bis 0,3 m³/m²·h am Kopf und 0,03 bis 1,0 m³/m²·h im Abtriebsteil der Kolonne erreicht. Überraschenderweise wurde gefunden, daß auch bei so niedrigen Flüssigkeitsbelastungen, die für eine optimale Trennleistung erforderliche vollständige Benetzung der Metallpackungen sichergestellt ist. Durch diese geringe Berieselungsdichte wird die Gasbelastung in der Kolonne und damit der Druckverlust außerordentlich gering.

Zur Erreichung einer optimalen Trennleistung ist jedoch nicht nur eine hohe Abtropfstellenzahl sondern auch die Anordnung der Verteiler im Bezug auf die Packungselemente wichtig.

Eine Lage einer Gewebepackung besteht im allgemeinen aus einer Vielzahl üblicherweise einzelner, 170 mm hoher Gewebelagen. Jede Packungslage wird beim Einbau um jeweils 90° im Bezug auf die vorhergehende eingebaut. Die Anordnung der Verteiler erfolgt ebenfalls um 90° gedreht in Bezug auf das direkt unter den Verteilern befindliche Packungselement, bzw. die dort befindliche Packungslage.

Die Flüssigkeit breitet sich nun auf einer dieser Gewebelagen in einem bestimmten Winkel aus. Nach einer von dem Ausbreitungswinkel und dem Abstand zweier Abtropfstellen abhängigen Einlauflänge hat sich ein gleichmäßiger Film über einer Gewebelage ausgebildet.

Eine optimale Ausnutzung der Packung, d.h. eines schellstmögliche Verteilung der Flüssigkeit auf allen Gewebelagen erzielt man, wenn man an dieser Stelle die Packung um 90° dreht.

Erfindungsgemäß werden daher unterhalb der Flüssigkeitsverteiler 2 oder mehrere Packungselemente mit einer Höhe von nur 20 bis 100 mm, vorzugsweise 25 bis 50 mm, insbesondere 35 bis 45 mm eingesetzt, deren Gewebelagen jeweils um 90° zueinander gedreht sind. Durch das Trennen der Packung in Elemente mit geringerer Höhe kann eine schnellstmögliche Verteilung und damit eine optimale Ausnutzung der Packung zum Trennen erzielt werden. Bei der herkömmlichen Packungsanordnung dagegen beträgt die Einlauflänge ca. 340 mm, was bedeutet, daß bei einer Packungshöhe von 2 Metern ca. 17 % der Packung nicht voll für die eigentliche Trennoperation genutzt werden.

Gemäß Merkmal e) soll die Rektifikation praktisch unter Luftausschluß durchgeführt werden.

Laborversuche haben gezeigt, daß es z.B bei VEA bei den für die Rektifikation benötigten hohen Temperaturen schon bei geringsten Leckagen in der Destillationseinrichtung zu einer Dunkelfärbung des Produktes kommt, die Aufgrund der hohen Qualitätsanforderungen nicht toleriert werden kann. Die Verwendung von neu entwickelten besonders hochwertigen Dichtungsmaterialien, wie Helicoflex® der Firma Cefilac, zur Abdichtung von Flanschen und/oder Öffnungen für Geräte zur Prozeßüberwachung ist daher unbedingt notwendig. Zur Abdichtung von Flanschen verwendet man mit besonderem Vorteil sogenannte Schweißlippendichtungen, wie sie beispielsweise in den deutschen Patentschriften DE 27 10 859, DE 39 12 478 oder DD 44 07 728 beschrieben sind.

Wie bereits dargestellt, zirkulieren in den erfindungsgemäßen Feinvakuumrektifikationskolonnen nur geringe Stoffströme. Daher führt jeder Wärmeverlust sofort zu wilder, d.h. unkontrollierter Kondensation an der Kolonnenwand, welche die Trennleistung der Kolonne reduziert. Die Verhinderung eines Wärmeaustausches durch die Kolonnenwand kann am besten durch eine Kombination aus Isolation und Schutzbeheizung der Kolonne gewährleistet werden.

Die technische Ausführung einer solchen Schutzbeheizung wird mit Vorteil praktisch auf folgende Weise realisiert: Auf einer ersten Isolierschicht auf dem Kolonnenmantel wird ein Blechmantel angebracht. Dieser Blechmantel wird erneut isoliert. Auf diese Isolierschicht wird dann ein weiterer Blechmantel und die Heizung aufgebracht und diese schließlich nach außen isoliert. Die Regelung der Heizung erfolgt dann derart, daß die Temperaturdifferenz zwischen dem Kolonnenmantel und dem ersten Blechmantel Null beträgt.

Für die erfindungsgemäße Feinreinigung von VEA ist es notwendig, daß man bei der Rektifikation mit Kopfdrucken von 0,2 bis 1 mbar, vorzugsweise 0,5 bis 1 mbar und mit Sumpfdrucken von 1 bis 4 mbar, vorzugsweise 1,5 bis 3,5 mbar, insbesondere 2 bis 3 mbar, arbeitet.

Bei der erfindungsgemäßen Rektifikation von VEA werden die Pakkungskolonnen mit einer Flüssigkeitsbelastung zwischen 0,03 und 0,3 m³/m²·h im Verstärkungsteil der Kolonne und mit einer Flüssigkeitsbelastung zwischen 0,03 und 1,0 m³/m²·h im Abtriebsteil der Kolonnen betrieben.

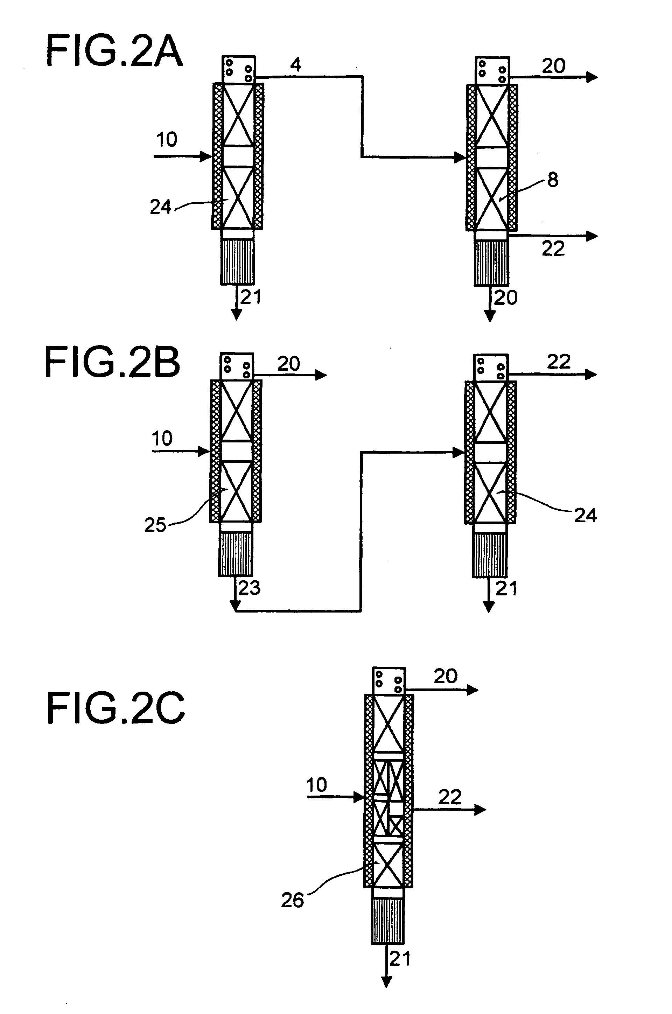

Zur Abtrennung von färbenden leichter siedenden Verunreinigungen und höher siedenden Verunreinigungen sind im allgemeinen und auch beim VEA 2 Kolonnen notwendig. Die Figuren 2a und 2b stellen schematisch 2 mögliche Destillationskonzepte für die Rektifikation von VEA in 2 Packungskolonnen dar. Hierin bedeuten

| | |
|---|---|
| 10 | Zulauf für Roh-Vitamin-E-acetat |
| 20 | Ablauf für Feed-Qualität |
| 21 | Ablauf für Feed-Qualität + Rückstand |
| 4 | Ablauf für Destillat |
| 22 | Ablauf für Food bzw. Pharma-Qualität, d.h. Reinheit > 99 % |
| 23 | Sumpf-Strom |
| 24 | Rektifikation zur Abtrennung der Schwersieder |
| 25 | Rektifikation zur Abtrennung der Leichtsieder. |

Man kann aber auch äquivalente Destillationseinrichtungen, wie eine sogenannte Trennblechkolonne verwenden. Unter einer Trennblechkolonne versteht man allgemein eine Kombination von 2 getrennten Kolonnenpackungen innerhalb von einem äußeren Kolonnenmantel, an dem sich Zulauf und Seitenabzug befinden.

Eine solche Trennblechkolonne ist schematisch in Figur 2c dargestellt. Hierin haben 10, 20, 21 und 22 die gleiche Bedeutung wie in den Figuren 2a und 2b und 26 bedeutet Trennblechkolonne.

Gegenstand der Erfindung ist auch die Verwendung der Packungskolonne für die Feinreinigung von verunreinigtem Vitamin-E-acetat oder Tocopherolen durch Rektifikation im Druckbereich zwischen 0,2 und 2 mbar.

Es handelt sich dabei um Packungskolonnen mit Abtriebsteil und Verstärkungsteil enthaltend Metallgewebepackungen mit geordneter Struktur, die jeweils um 90° zueinander gedreht sind zum Rektifizieren von Gemischen hochsiedender luft- und/oder temperaturempfindlicher Substanzen, die eine hohe Trennleistung erfordern, bei Kopfdrücken von 0,2 bis 2 mbar, die dadurch gekennzeichnet sind,
a) daß sie als Flüssigkeitsverteiler sogenannte Kanalverteilermit gleich oder mehr als 500 Abtropfstellen/m², vorzugsweise 900 bis 1200 Abtropfstellen/m² pro m² enthalten,
b) die Kanäle der Flüssigkeitsverteiler im Winkel von etwa 90° um die Kolonnenachse verdreht zu den Gewebelagen der direkt unter dem Verteiler befindlichen Packungselemente angeordnet sind,
c) daß sie unterhalb der Flüssigkeitsverteilung 2 oder mehrere Packungselemente mit einer Höhe von nur 20 bis 100 mm Höhe enthalten, deren Gewebelagen jeweils um 90° um die Kolonnenachse zueinander gedreht sind,
d) daß sie eine Kombination aus Isolierung und Schutzbeheizung enthalten, durch die gewährleistet ist, daß während der Rektifikation praktisch kein Wärmeverlust durch die Kolonnenwand stattfinden kann und
e) daß Dichtungen und Flansche so ausgestaltet sind, daß praktisch ein Luftausschluß gewährleistet ist.

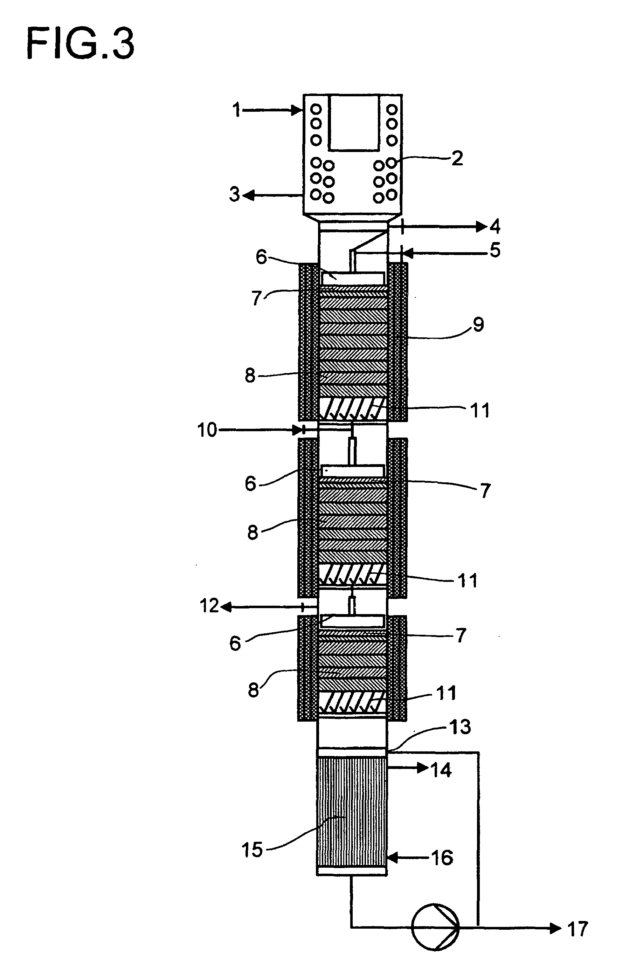

Eine erfindungsgemäß für die Rektifikation von VEA einsetzbare Packungskolonne ist in Figur 3 schematisch dargestellt.

### Hierin bedeuten:

- 1: Zulauf für Kühlmedium
- 2: Kondensator
- 3: Ablauf für Kühlmedium
- 4: Destillat
- 5: Rücklauf
- 6: Kanal-Flüssigkeitsverteiler mit gleich oder mehr als 500 Abtropfstellen/m²
- 7: Packungselemente mit einer Höhe von 20 bis 100 mm
- 8: Packungselemente mit einer Höhe von ca. 170 mm
- 9: Schutzheizung
- 10: Zulauf für Roh-VEA
- 11: Flüssigkeitssammler
- 12: Seitenabzug
- 13: Dichtungselement
- 14: Ablauf für Heizmedium (Ausgang)
- 15: Rieselfilmverdampfer
- 16: Zulauf für Heizmedium (Eingang)
- 17: Sumpfabzug.

Mit Hilfe der erfindungsgemäßen Packungskolonnen und des erfindungsgemäßen Verfahrens ist es möglich Gemische hochsiedender luft- und/oder temperaturempfindlicher Substanzen, die eine hohe Trennleistung erfordern, wie Roh-VEA oder Gemische von Tocopherolen mit Verunreinigungen, auch im Feinvakuum, d.h. bei Drucken von 0,2 bis 2 mbar, vorzugsweise 0,5 bis 1 mbar in Kolonnen mit druckverlustarmen Gewebepackungen mit sehr guten Destillationsausbeuten zu rektifizieren.

Beispielhafte Beschreibung des Verfahrens zur Reinigung von Roh-Vitamin-E-acetat nach dem Destillationskonzept gemäß Figur 2a.

Entgastes, synthetisch hergestelltes Roh-Vitamin-E-acetat mit einem Gehalt von etwa 96 % VEA wird bei einer Temperatur zwischen 200 und 250 Grad Celsius durch den Zulauf 10 in die Mitte der ersten Rektifikationskolonne mit einer Packungshöhe von 3 bis 5 m vom Typ Sulzer BX oder Montz A3 eingetragen. Die Flüssigkeit wird am Kopf und am Zulauf der Kolonne mit den neu entwickelten Hochleistungs-Kanalverteilern 6 gleichmäßig über den Kolonnenquerschnitt verteilt. Unterhalb der neu entwickelten Kanalverteiler befinden sich die Packungselemente 7 mit einer Höhe von nur 20 bis 100 mm Höhe. Die Gewebelagen der direkt unter den Kanalverteilern 6 angeordneten Packungselemente sind zu den Kanälen der Flüssigkeitsverteiler im Winkel von 90° verdreht. Die Kolonne ist mit einer Schutzheizung 9 ausgestattet und wird adiabatisch betrieben. Flansche und Stutzen sind mit Schweißlippendichtungen bzw. hochwertigen Metalldichtungen versehen.

Der Kopfdruck der Kolonne beträgt 0,5 bis 1 mbar. Das Rücklaufverhältnis beträgt 2 bis 4. Die in der Kolonne zurücklaufende Flüssigkeit hat eine Temperatur zwischen 170 und 220 Grad Celius. Am Sumpf der Kolonne wird 5 bis 20 % des Zulaufstromes entnommen.

Das am Kopf der ersten Kolonne entnommene Produkt mit einer Temperatur von 200 bis 250°C in die Mitte der zweiten Rektifikationskolonne mit 3 bis 5 m Packungshöhe vom Typ Sulzer BX oder Montz A3 gefördert. Die Flüssigkeit wird am Kopf und am Zulauf mit den neu entwickelten Hochleistungs-Kanalverteilern über den Kolonnenquerschnitt verteilt. Auch diese Kolonne ist mit einer Schutzheizung ausgerüstet und wird adiabatisch betrieben. Flansche und Stutzen sind mit Schweißlippendichtungen bzw. hochwertigen Metalldichtungen versehen.

Der Kopfdruck der Kolonne beträgt ca. 0,5 mbar. Das Rücklaufverhältnis beträgt 5 bis 15. Die in die Kolonne zurücklaufende Flüssigkeit hat eine Temperatur zwischen 170 bis 220°C. Am Kopf und im Sumpf der Kolonne werden 5 bis 20 % des Zulaufstromes entnommen. Das im Seitenabzug unmittelbar oberhalb des Verdampfers der zweiten Kolonne entnommene, Vitamin-E-acetat ist nahezu farblos (Farbzahl kleiner als 2) und weist Pharma-Qualität auf mit einer Reinheit von mehr als 99 %. Die Ausbeute an so reinem Produkt beträgt etwa 50 % der Theorie.

In einem Dünnschichtverdampfer wird bei einem Druck zwischen 1 bis 5 mbar der Rückstand von der Sumpfentnahme der ersten Rektifikationskolonne abgetrennt. Das so gewonnene Destillat kann wie das Kopf- und Sumpfprodukt der zweiten Kolonne als Vitamin-E-acetat für Viehfutterzwecke (feed-Qualität) verkauft werden. Diese Produkte enthalten im allgemeinen 90 bis 99 % VEA. Die gesamte Destillationsausbeute an feed- und Pharma-VEA beträgt zwischen 95 und 98 %.

## Patentansprüche

1. Verfahren zur Gewinnung von reinem Vitamin-E-acetat oder reinen Tocopherolen aus Gemischen durch Rektifikation in einer Stoffaustauschkolonne, die Metallgewebepackungen mit geordneter Struktur enthält, bei einem Kopfdruck von 0,2 bis 2 mbar und in der
a) die Flüssigkeitsverteilung mit Kanalverteilern mit gleich oder mehr als 500 Abtropfstellen pro m² vorgenommen wird,
b) in der die Kanäle der Flüssigkeitsverteiler im Winkel von etwa 90° um die Kolonnenachse verdreht zu den Gewebelagen der direkt unter diesen Verteilern befindlichen Packungselemente angeordnet sind,
c) in der sich unterhalb der Flüssigkeitsverteiler 2 oder mehrere Packungselemente mit einer Höhe von nur 20 bis 100 mm Höhe befinden, deren Gewebelagen jeweils um 90° um die Kolonnenachse zueinander gedreht sind,
d) daß die Kolonne so gestaltet ist, daß während der Rektifikation praktisch kein Wärmeaustausch durch die Kolonnenwand stattfinden kann,
e) die Kolonne so gestaltet ist, daß praktisch unter Luftausschluß gearbeitet werden kann und
f) der Druckverlust pro Trennstufe durch eine erniedrigte Einstellung der Flüssigkeitsbelastung soweit erniedrigt wird, daß die im Sumpf auftretenden Temperaturen unterhalb des Zersetzungsbereichs von Vitamin-E-acetat erniedrigt werden.

2. Verfahren gemäß Anspruch 1, **dadurch gekennzeichnet, daß** in f) die Flüssigkeitsbelastung minimal so eingestellt wird, daß noch eine vollständige Benetzung der Metallpackung sichergestellt ist.

3. Verfahren gemäß Anspruch 1 oder 2, **dadurch gekennzeichnet, daß** die Flüssigkeitsverteilung gemäß a) mit Kanalverteilern mit 900 bis 1200 Abtropfstellen pro m² vorgenommen wird.

4. Verfahren gemäß einem der Ansprüche 1 bis 3, **dadurch gekennzeichnet, daß** der Luftausschluß gemäß e) durch Verwendung besonders hochwertiger Dichtungsmaterialien oder durch den Einsatz von sogenannten Schweißlippendichtungen gewährleistet wird.

5. Verfahren gemäß einem der Ansprüche 1 bis 4, **dadurch gekennzeichnet, daß** man in d) durch eine Kombination aus Isolation und Schutzbeheizung der Kolonne gewährleistet, daß praktisch kein Wärmeverlust durch die Kolonnenwand auftreten kann.

6. Verfahren gemäß einem der Ansprüche 1 bis 5, **dadurch gekennzeichnet, daß** man in c) als Packungselement Gewebepackungen verwendet, bei denen der Neigungswinkel der Zahnung der einzelnen Gewebelagen der Packung gegen die Kolonnenachse 0 bis 25° beträgt.

7. Verfahren gemäß Anspruch 6, **dadurch gekennzeichnet, daß** der Neigungswinkel 3 bis 10° beträgt.

8. Verfahren gemäß einem der Ansprüche 1 bis 7, **dadurch gekennzeichnet, daß** man mit Kopfdrücken von 0,5 bis 1 mbar und mit Sumpfdrücken von 1 bis 4 mbar arbeitet.

9. Verfahren gemäß einem der Ansprüche 1 bis 8, **dadurch gekennzeichnet, daß** in Merkmal f) von Anspruch 1 dir Kolonnen mit einer Flüssigkeitsbelastung zwischen 0,03 und 0,3 m³/m²·h am Kopf und einer Flüssigkeitsbelastung zwischen 0,03 und 1,0 m³/m²·h im Abriebsteil betrieben werden.

10. Verwendung von Packungskolonnen mit Abtriebsteil und Verstärkungsteil enthaltend Metallgewebepackungen mit geordneter Struktur (8), die jeweils um 90° zueinander gedreht sind, wobei
a) sie als Flüssigkeitsverteiler sogenannte Kanalverteiler mit gleich oder mehr als 500 Abtropfstellen/m² (6) enthalten,
b) die Kanäle der Flüssigkeitsverteiler im Winkel von etwa 90° um die Kolonnenachse verdreht zu den Gewebelagen der direkt unter diesen Verteilern befindlichen Packungselemente (7) angeordnet sind,
c) sie unterhalb der Flüssigkeitsverteilung 2 oder mehrere Packungselemente mit einer Höhe von nur 20 bis 100 mm Höhe (7) enthalten, deren Gewebelagen jeweils um 90° um die Kolonnenachse zueinander gedreht sind,
d) sie eine Kombination aus Isolierung und Schutzbeheizung (9) enthalten, durch die gewährleistet ist, daß während der Rektifikation praktisch kein Wärmeverlust durch die Kolonnenwand stattfinden kann und
e) Dichtungen und Flansche so ausgestaltet sind, daß in den Kolonnen praktisch ein Luftausschluß gewährleistet ist,
für die Feinreiniugng von verunreinigtem Vitamin-E-acetat oder Tocopherolen durch Rektifikation bei Kopfdrücken von 0,2 mbar bis 2 mbar.

## Claims

1. A process for isolating pure vitamin E acetate or pure tocopherols from mixtures, by rectification in a mass transfer column which comprises metal cloth packings with ordered structure, at an overhead pressure of from 0.2 to 2 mbar, and in which
a) the liquid distribution is undertaken with channel distributors with 500 or more drip points per m²,
b) in which the channels of the liquid distributors are arranged at an angle of about 90°, rotated about the column axis, to the cloth layers of the packing elements located immediately below these distributors,
c) in which 2 or more packing elements which have a height of from 20 to 100 mm and whose cloth layers are in each case rotated by 90° with respect to one another about the column axis, are located below the liquid distributors,
d) the column is designed so that virtually no heat exchange through the column wall can take place during the rectification,
e) the column is designed so that it is possible to operate virtually with exclusion of air, and
f) the pressure drop per separation stage is decreased by a decreased setting of the liquid flow rate to such an extent that the temperatures occurring in the bottom are decreased below the decomposition range for vitamin E acetate.

2. A process as claimed in claim 1, wherein the minimal setting of the liquid flow rate in f) is such that complete wetting of the metal packing is still ensured.

3. A process as claimed in claim 1 or 2, wherein the liquid distribution in a) is undertaken with channel distributors with 900 to 1200 drip points per m².

4. A process as claimed in any of claims 1 to 3, wherein the exclusion of air in e) is ensured by using particularly high quality sealing materials or by using welded lip seals.

5. A process as claimed in any of claims 1 to 4, wherein a combination of insulation and protective heating of the column in d) ensures that virtually no heat loss through the column wall can occur.

6. A process as claimed in any of claims 1 to 5, wherein the cloth packings used as packing element in c) have an angle of inclination of the serration of the individual cloth layers of the packing to the column axis of from 0 to 25°.

7. A process as claimed in claim 6, wherein the angle of inclination is from 3 to 10°.

8. A process as claimed in any of claims 1 to 7, wherein overhead pressures of from 0.5 to 1 mbar and bottom pressures of from 1 to 4 mbar are used.

9. A process as claimed in any of claims 1 to 8, wherein the columns are operated with a liquid flow rate of from 0.03 to 0.3 m³/m².h overhead and with a liquid flow rate of from 0.03 to 1.0 m³/m²·h in the stripping part in feature f) of claim 1.

10. The use of packing columns with a stripping part and concentrating part comprising metal cloth packings with ordered structure (8), each of which are rotated by 90° with respect to one another, where
a) they comprise channel distributors with 500 or more drip points/m² (6) as liquid distributors,
b) the channels of the liquid distributors are arranged at an angle of about 90°, rotated about the column axis, to the cloth layers of the packing elements (7) located immediately below these distributors,
c) they comprise below the liquid distribution 2 or more packing elements which have a height of from 20 to 100 mm (7) and whose cloth layers are in each case rotated by 90° with respect to one another about the column axis,
d) they comprise a combination of insulation and protective heating (9), which ensures that virtually no heat loss through the column wall can take place during the rectification, and
e) the seals and flanges are designed so that virtually exclusion of air in the columns is ensured
for final purification of impure vitamin E acetate or tocopherols by rectification at overhead pressures of from 0.2 to 2 mbar.

## Revendications

1. Procédé de production d'acétate de vitamine E pur ou de tocophérols purs à partir de mélanges, par rectification dans une colonne de transfert de matière, qui contient des garnissages en tissu métallique à structure ordonnée, à une pression de tête de 0,2 à 2 mbars, et dans laquelle
a) la répartition de liquide est effectuée par des répartiteurs à canaux présentant 500 points d'égouttage par m² ou davantage,
b) les canaux des répartiteurs de liquide sont agencés tournés sous un angle d'environ 90° autour de l'axe de la colonne par rapport aux couches de tissu des éléments de garnissage situés directement en dessous de ces répartiteurs,
c) en dessous des répartiteurs de liquide se trouvent deux ou plusieurs éléments de garnissage ayant une hauteur de seulement 20 à 100 mm, dont les couches de tissu sont tournées l'une par rapport à l'autre chaque fois de 90° autour de l'axe de la colonne,
d) la colonne étant conformée de façon que, pendant la rectification, pratiquement aucun échange de chaleur ne puisse avoir lieu au travers de la paroi de colonne,
e) la colonne étant conformée de façon à pouvoir fonctionner pratiquement en absence d'air, et
f) la perte de pression par étage séparateur est abaissée par un ajustement diminué de la charge de liquide dans la mesure où les températures apparaissant dans le fond de colonne sont abaissées en dessous de l'intervalle de décomposition d'acétate de vitamine E.

2. Procédé suivant la revendication 1, **caractérisé en ce que**, dans f), la charge de liquide est ajustée de manière minimale de façon à assurer encore un mouillage complet du garnissage métallique.

3. Procédé suivant l'une des revendications 1 et 2, **caractérisé en ce que** la répartition de liquide selon a) est effectuée avec des répartiteurs à canaux comportant 900 à 1200 points d'égouttage par m².

4. Procédé suivant l'une des revendications 1 à 3, **caractérisé en ce que** l'absence d'air conformément à e) est garantie par utilisation de matériels d'étanchéification de particulièrement grande valeur ou par la mise en oeuvre de ce que l'on appelle des joints à lèvres de soudure.

5. Procédé suivant l'une des revendications 1 à 4, **caractérisé en ce que**, dans d), par une combinaison d'isolation et de chauffage de protection de la colonne, on garantit qu'il ne puisse apparaître pratiquement aucune perte de chaleur à travers la paroi de colonne.

6. Procédé suivant l'une des revendications 1 à 5, **caractérisé en ce que**, dans c), on utilise, comme élément de garnissage, des garnissages en tissu dans lesquels l'angle d'inclinaison de la denture des différentes couches de tissu du garnissage par rapport à l'axe de colonne est de 0 à 25°.

7. Procédé suivant la revendication 6, **caractérisé en ce que** l'angle d'inclinaison est de 3 à 10°.

8. Procédé suivant l'une des revendications 1 à 7, **caractérisé en ce qu'**on opère avec des pressions de tête de 0,5 à 1 mbar et des pressions de fond de colonne de 1 à 4 mbars.

9. Procédé suivant l'une des revendications 1 à 8, **caractérisé en ce que**, dans la particularité f) de la revendication 1, les colonnes sont mises en service avec une charge de liquide comprise entre 0,03 et 0,3 m³/m²·h à la tête et une charge de liquide comprise entre 0,03 et 1,0 m³/m²·h dans la partie de stripage.

10. Utilisation de colonnes à garnissage comportant une partie de stripage et une partie de concentration contenant des garnissages en tissu métallique à structure ordonnée (8), qui chacune sont tournées l'une par rapport à l'autre de 90°, dans laquelle
a) elles contiennent, comme répartiteurs de liquide, ce que l'on appelle des répartiteurs à canaux présentant 500 points d'égouttage/m² (6) ou davantage,
b) les canaux des répartiteurs de liquide sont agencés d'une manière tournée sous un angle d'environ 90° autour de l'axe de la colonne par rapport aux couches de tissu des éléments de garnissage (7) situés directement en dessous de ces répartiteurs,
c) elles contiennent, en dessous de la répartition de liquide 2 ou plusieurs éléments de garnissage ayant une hauteur de seulement 20 à 100 mm (7), dont les couches de tissu sont tournées respectivement l'une par rapport à l'autre de 90° autour de l'axe de colonne,
d) elles contiennent une combinaison d'isolation et de chauffage de protection (9) par laquelle il est garanti que, pendant la rectification, pratiquement aucune perte de chaleur ne peut avoir lieu à travers la paroi de colonne, et
e) des éléments d'étanchéité et des brides sont conçus de façon que, dans les colonnes, une quasi-absence d'air soit garantie, pour l'épuration fine d'acétate de vitamine E ou de tocophérols contaminés, par rectification à des pressions de tête de 0,2 mbar à 2 mbars.
